Europäisches Patentamt

European Patent Office

Office européen des brevets

(19)

(11) EP 0 488 835 B1

(12) **FASCICULE DE BREVET EUROPEEN**

(45) Date de publication et mention
de la délivrance du brevet:
**08.03.2000 Bulletin 2000/10**

(51) Int. Cl.[7]: **A61F 2/16**

(21) Numéro de dépôt: **91402876.6**

(22) Date de dépôt: **28.10.1991**

(54) **Implant intraoculair multifocale**

Multifokale intraokulare Implantat

Multifocal intraocular implant

(84) Etats contractants désignés:
**BE CH DE DK ES GB GR IT LI LU NL**

(30) Priorité: **31.10.1990 FR 9013753**

(43) Date de publication de la demande:
**03.06.1992 Bulletin 1992/23**

(73) Titulaire: **CORNEAL INDUSTRIE**
**F-74370 Pringy (FR)**

(72) Inventeurs:
• **Bos, Gilles**
**F-74940 Annecy le Vieux (FR)**
• **Lesauvage, Pierre**
**F-74000 Annecy (FR)**

(74) Mandataire: **Dronne, Guy et al**
**Cabinet Beau de Loménie**
**158, rue de l'Université**
**75340 Paris Cédex 07 (FR)**

(56) Documents cités:
**EP-A- 0 107 444        EP-A- 0 201 231**
**WO-A-89/02251        WO-A-90/00889**
**US-A- 1 910 483        US-A- 1 955 047**
**US-A- 4 636 211**

**Description**

La présente invention concerne le domaine technique général des lentilles multifocales et, notamment, bifocales permettant la vision alternée de près et de loin, selon le choix effectué par l'observateur.

La présente invention concerne, plus particulièrement mais non exclusivement, un implant intra-oculaire multifocal et, notamment, bifocal, de type réfractif, présentant des zones réfractives de courbures différentes.

De tels implants artificiels sont destinés à remplacer les cristallins de patients à la suite d'accidents ou d'opacifications du cristallin. Ces implants doivent répondre à diverses contraintes et divers critères, afin, non seulement d'être tolérés sur le plan physiologique par l'organisme, mais encore afin d'assurer au patient une vision comparable en toutes conditions lumineuses à celle obtenue avec un cristallin naturel.

Le premier critère fondamental auquel doit satisfaire un implant multifocal est, bien évidemment, celui de posséder au moins deux focales, afin de permettre au patient une vision de près et de loin.

Le second impératif à satisfaire est lié à la variation de dimension de la pupille sous l'influence des conditions également variables de l'éclairement lumineux. En effet, la dilatation ou la rétractation de la pupille, selon l'intensité lumineuse, modifie la surface d'éclairement totale de l'implant. Bien évidemment, à chacune de ces zones d'éclairage doit correspondre, pour le patient, une possibilité théorique de vision bifocale, c'est-à-dire de loin et de près.

Il convient également de noter que, d'une manière générale, on considére que la vision de loin correspond aux forts éclairements (pupille rétractée ou étroite), ainsi qu'aux faibles luminosités (pupille dilatée au maximum). En revanche, il est couramment admis que la vision de prés correspond à une intensité lumineuse intermédiaire associée à une pupille moyennement dilatée.

Le second impératif à respecter consiste donc à fournir au patient la possibilité d'utiliser, de manière privilégiée, la bonne focale en fonction des conditions d'éclairement instantané, à savoir une vision de près privilégiée en condition d'éclairement intermédiaire et une vision de loin en condition de fort et faible éclairement.

Le troisième impératif à satisfaire est partiellement lié au second, dans la mesure où il consiste à fournir au patient, quelle que soit la variation de l'éclairement rétinien, pour chaque focale, la possibilité de voir selon différentes focales. Ceci implique de tenir compte de l'ouverture de la pupille et de ses contraintes dimensionnelles de dilatation et de rétractation maximale.

Il a déjà été proposé pour remédier en partie aux problèmes évoqués précédemment, de réaliser, dans un autre domaine technique que celui de la réalisation des implants intra-oculaires, des lentilles de contact à deux zones de vision différenciées, de près et de loin.

Le brevet **US-A-4 636 049** ou le brevet WO 89/02251 décrivent une lentille de contact de ce type, munie d'une zone centrale circulaire de vision de prés et d'une zone périphérique et annulaire externe, de vision de loin. La zone centrale de vision de près possède une superficie qui est sensiblement équivalente à la moitié de la surface de la pupille, lorsque celle-ci est soumise à des conditions d'éclairement intermédiaire permettant la lecture.

Une telle lentille permet, bien évidemment, une vision bifocale, mais ne privilégie la vision de prés que dans les conditions d'éclairement intermédiaires. Avec ce type de lentilles, il n'est, en effet, jamais possible d'obtenir une possibilité de vision selon une double focale, quelques soient les variations des conditions d'éclairement, puisqu'en condition de fort éclairement il n'est pas possible, pour le patient, de voir de loin. Inversement, en cas de faible éclairement, et donc d'ouverture pupillaire maximale, la vision de près est quasiment inexistante, voire impossible.

On connaît également, d'après la demande internationale **PCT/WO/90-00 889**, un implant oculaire multifocal comportant trois zones de vision différenciées consistant en une zone centrale circulaire réservée à la vision de loin, entourée de deux zones annulaires concentriques et circulaires à vision alternée, respectivement, de près et de loin. Un tel implant, de par la disposition alternée de ses trois zones de vision, privilégie la vision de loin dans la plupart des conditions d'éclairement.

Un tel implant ne résoud pas parfaitement le problème lié à la vision de loin aux forts éclairements. En effet, soit la zone centrale a un faible diamètre, inférieur à 1,7 mm par exemple et la vision de loin n'est, dans ce cas, pas prépondérante en cas de fort éclairement. Une telle particularité présente l'inconvénient notable de ne pas permettre, en cas de conduite de nuit par exemple, à un conducteur de conserver une vision de loin en cas d'éblouissement. La zone centrale peut encore présenter un diamètre plus important, proche, par exemple, de 2 mm, mais alors, en cas de forte rétractation de la pupille, la vision de près est quasiment inexistante et l'on ne peut alors parler de vision bifocale. Un tel implant intra-oculaire ne présente donc pas tous les aspects de sécurité de vision sous toutes les conditions d'éclairement susceptibles de survenir et demeure d'une utilisation spécifique réservée à la vision de loin.

On connaît enfin, d'après le brevet **EP-A-0 140 063**, un implant multizones comprenant une zone centrale et circulaire de vision de près entourée de zones de vision, alternativement de loin et de près, et concentriques à la zone centrale.

Dans un tel implant, les surfaces respectives de chacune des zones de vision différenciées, de prés ou de loin, sont égales, ce qui conduit à un implant permettant, certes une vision bifocale selon la plupart des conditions d'éclairement, mais ne privilégiant aucune focale de vision en relation avec une ou des conditions d'éclairement spécifiques. En particulier, lors d'une vision en condition de faible éclairement et, notamment la nuit, la vision de prés est équivalente à la vision de loin, alors même qu'en théorie, pour des raisons de sécurité, la vision de nuit devrait être privilégiée.

Un tel implant est d'ailleurs plus particulièrement conçu pour être utilisé en liaison avec des traitements médicamenteux ou microchirurgicaux fixant ou maintenant le diamètre de la pupille a ou dans des limites dimensionnelles contrôlées.

L'objet de l'invention vise donc à remédier aux inconvénients des lentilles multifocales évoqués ci-dessus et à fournir une lentille multifocale, et notamment un implant intra-oculaire bifocal de type réfractif, apte à fournir une possibilité de vision bifocale, quelles que soient les conditions d'éclairement, et privilégiant la vision de loin en condition d'éclairement maximum.

Un autre objet de l'invention vise à fournir une lentille multifocale, et notamment un implant intra-oculaire bifocal réfractif, apte à fournir une possibilité de vision bifocale quelles que soient les conditions d'éclairement et apte à privilégier, dans des conditions d'éclairement intermédiaire, la vision de près au détriment de la vision de loin.

Un objet supplémentaire de l'invention est de fournir une lentille multifocale, et notamment un implant intra-oculaire bifocal réfractif, apte à fournir, en toutes conditions d'éclairement, une vision bifocale et à privilégier la vision de loin en conditions d'éclairement minimum.

Les buts assignés à l'invention sont atteints à l'aide d'une lentille multifocale, et notamment un implant intra-oculaire bifocal rétractif comportant un bloc optique transparent consistant en au moins deux zones concentriques de correction de vision, chaque zone étant, à partir de la zone centrale réservée à la vision de près, alternativement, une zone de vision de loin et de loin, caractérisée en ce que les surfaces respectives de chacune des zones sont inégales et choisies de telle manière que, dans des conditions d'éclairement maximum, la surface consacrée à la vision de loin est supérieure à la moitié de la surface totale éclairée.

Diverses autres caractéristiques ressortent de la description faite ci-dessous en référence aux dessins annexés qui montrent, à titre d'exemples non limitatifs, des formes de réalisation de l'objet de l'invention.

La **fig. 1** représente une vue schématique d'un globe oculaire selon une coupe transversale partielle.

La **fig. 2** représente une vue de face d'une lentille multifocale selon l'invention.

La **fig. 3** représente une vue de profil latérale d'une lentille multifocale conforme à l'invention.

La **fig. 4** représente un graphe montrant, pour une lentille donnée, conforme à l'invention, la répartition privilégiée de la vision de près et de loin selon la variation du diamètre d'ouverture de la pupille.

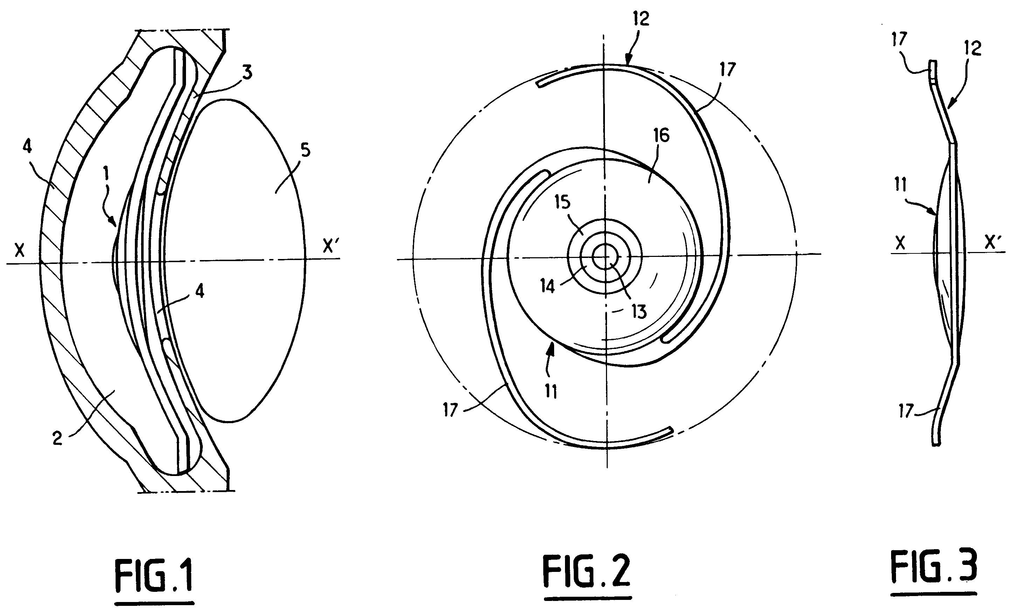

La **fig. 1** montre, de manière schématique, selon une coupe transversale partielle, la partie antérieure du globe oculaire d'un être humain à l'intérieur duquel un implant intra-oculaire **1** est mis en place, dans la chambre antérieure **2** par exemple. Cette dernière est délimitée, de manière classique, par l'iris **3** percé d'un orifice **4** constituant la pupille. En arrière de la pupille se trouve le cristallin **5**. L'implant intra-oculaire **1** peut, bien évidemment, être disposé différemment et, par exemple, dans la chambre postérieure du globe oculaire.

L'implant intra-oculaire **1** selon l'invention, tel que représenté aux **fig. 2** et **3**, est constitué d'un bloc optique central **11**, mono ou multi-matériaux, et d'une partie haptique **12** dont la fonction est d'assurer l'immobilisation de l'implant **1** à l'intérieur du globe oculaire. Le bloc optique **11** peut être constitué, de manière classique, en un matériau de type plastique utilisé communément en optique intra-oculaire et présentant des caractéristiques de transparence et d'inertie chimique vis-à-vis des tissus et liquides du globe oculaire.

De tels matériaux sont bien connus et peuvent être, par exemple, du silicone, des polycarbonates ou des polymétaphylates ou polymétacrylates par exemple. Le bloc optique **11** selon l'invention présente une forme générale sensiblement circulaire, d'épaisseur maximale voisine de 0,60 mm, et est constitué, avantageusement, d'au moins trois zones, et de préférence quatre, de vision différentielles concentriques à l'axe central **x-x′**.

L'implant **1** comporte une zone centrale **13**, constituée d'une partie circulaire dont la fonction optique est celle d'assurer une vision de près, et une zone **14**, immédiatement adjacente et jointive, en forme d'anneau. Le matériau, composant la zone **14** et/ou son rayon de courbure propre, sont choisis de manière à lui conférer des propriétés optiques de vision de loin. Avantageusement, l'implant comprend une zone annulaire **15**, immédiatement adjacente à la zone **14** et l'entourant sur toute sa périphérie et dont le matériau et/ou son rayon de courbure propre sont choisis de telle manière qu'elle assume une fonction optique de vision de près.

De manière préférentielle, il est également possible d'adjoindre une quatrième zone **16** aux propriétés optiques définies de vision de loin, comme cela est représenté aux **fig. 2** et **3**.

On obtient ainsi un implant intra-oculaire **1** constitué d'au moins deux zones de vision différenciées, et de préférence trois ou quatre, dont l'immobilisation dans le globe oculaire peut être assurée, de manière classique, par la partie haptique **12** comportant, par exemple, une paire d'anses courbes **17** solidaires de la périphérie de la zone terminale du bloc optique

11. Il est, bien évidemment, possible d'avoir recours à tout autre moyen d'immobilisation connu, du type collerette, ou de toute autre forme appropriée.

Les zones de vision différenciées et alternées de près ou de loin **13, 14, 15** et **16** définissent, respectivement, sur leurs faces antérieures destinées à recevoir le flux lumineux, une surface de réception des rayons lumineux traversant la pupille. Ces surfaces, respectivement référencées $s_1$, $s_2$, $s_3$ et $s_4$ en partant de la zone centrale **14**, sont inégales et choisies en fonction des plages de variations de dimensions de la pupille, elles-mêmes fonction des conditions de variation de l'éclairement lumineux.

On sait que les diamètres pupillaires varient selon plusieurs facteurs mais, essentiellement, en fonction de l'intensité lumineuse. Il est également bien connu de l'homme de l'art que l'on considére, généralement, trois plages principales d'intensité lumineuse correspondant aux conditions naturelles de vision de loin, de prés, puis à nouveau de loin, à savoir :

- les conditions maximales d'éclairement associées à une forte luminosité d'une intensité lumineuse d'environ 350 candella/m$^2$ et correspondant à une vision de loin,
- les conditions d'éclairement intermédiaire associées à une vision d'intérieur et de près correspondant à une intensité lumineuse d'environ 35 candella/m$^2$,
- les conditions minimales d'éclairement associées à une vision de nuit et de loin correspondant à environ 3,5 $10^{-1}$ et 3,5 $10^{-3}$ candella/m$^2$.

Ces valeurs n'ont, bien évidemment, aucun caractère absolu mais correspondent à des données couramment acceptées dans le domaine technique considéré.

On peut ainsi associer, à chacune de ces trois conditions naturelles de vision, une ouverture pupillaire dont la détermination est statique et d'autant plus faible que l'intensité lumineuse est faible. Les caractéristiques de variation de l'ouverture pupillaire sont variables selon les individus et, également, selon leur âge, les personnes âgées présentant, par exemple, une ouverture moyenne de la pupille qui tend à diminuer au fur et à mesure de leur vieillissement.

A titre indicatif, par exemple, la plage d'ouverture utile totale d'une personne âgée et, par exemple, de plus de 60 ans, est généralement comprise entre 2 et 5 mm. La plupart des études statistiques ont montré les corrélations suivantes qui permettent de déterminer des plages moyennes de variation d'ouverture pupillaire :

- en forte luminosité ou conditions d'éclairement maximum (vision de loin), le diamètre de la pupille varie entre 2 mm et 2,8 mm,
- en condition d'éclairement intermédiaire (vision de près), le diamètre de la pupille varie entre 2,8 mm et 3,5 mm,
- en condition d'éclairement minimum (vision de loin, le diamètre de la pupille varie entre 3, 5 mm et 5 mm.

En fonction de ces plages d'ouverture pupillaire, il s'avère donc possible, pour chacune des trois conditions d'éclairement précédemment définies, de déterminer les surfaces des zones $s_1$ à $s_3$ et, éventuellement, $s_4$, afin de satisfaire à une vision bifocale privilégiée. Dans le cas présent, il s'agit de privilégier la vision de loin en condition d'éclairement maximal et minimal et la vision de près en condition de vision intermédiaire, tout en permettant, pour chaque type de vision, une vision bifocale permettant à l'observateur de choisir, inconsciemment et indifféremment, une vision de près ou de loin.

Pour que de telles conditions soient remplies, il faut qu'en condition d'éclairement maximum et minimum, correspondant à une plage de variations théoriques et données de l'ouverture pupillaire, la surface consacrée à la vision de loin, c'est-à-dire la fraction de surface des zones $s_2$ et, éventuellement, $s_4$ éclairées soit supérieure à la moitié de la surface totale de l'implant éclairé. Dans une telle situation, l'observateur bénéficie, lorsqu'il choisit de voir de loin, d'une quantité ou intensité de lumière supérieure à celle impressionnant ou éclairant les zones **13** ou **15** réservées à la vision de près.

De la même façon, pour privilégier en conditions d'éclairement intermédiaire, la vision de prés correspondant à l'opération de lecture, la surface consacrée à la vision de près doit être supérieure à la moitié de la surface totale éclairée par l'intermédiaire de l'ouverture pupillaire. A titre d'exemple, le diamètre externe du bloc optique **11** est voisin de 7 mm.

Compte tenu des contraintes mentionnées ci-dessus, et du choix d'ouvertures pupillaires de références correspondant, par exemple, à 2, 2,8 et 3,5 mm, pour lesquelles la vision de près sera sensiblement équivalente à la vision de loin, il est possible de déterminer, par calcul arithmétique, les plages dimensionnelles théoriques et pratiques que doivent respecter chacune des zones **13**, **14**, **15** et, éventuellement, **16**.

En considérant la zone **13** de diamètre $d_1$, la zone **14** de diamètre $d_2$, la zone **15** de diamètre $d_3$ et la zone **16** de diamètre $d_4$, on peut montrer qu'en condition d'éclairement maximum (diamètre pupillaire proche de 2 mm), la détermination de $d_1$ et $d_2$ est indépendante de $d_3$. En condition d'éclairement maximum et intermédiaire, il est théoriquement nécessaire que :

$$1 \text{ mm} \leqq d_1 \leqq 1,4 \text{ mm}$$

et :

$$1,7 \text{ mm} \leqq d_2 \leqq 2,50 \text{ mm}$$

pour que la vision de loin soit privilégiée en condition d'éclairement maximum et pour que la vision de près soit privilégiée en condition d'éclairement intermédiaire. A partir des mêmes plages de valeurs dimensionnelles de $d_1$ et $d_2$ et dans le cas où l'implant comporte une troisième zone **15** de vision de près et, avantageusement, une quatrième zone **16** de vision de loin, il est nécessaire de respecter, en outre, la condition suivante :

$$2,70 \text{ mm} \leqq d_3 \leqq 3,3 \text{ mm}$$

pour qu'en conditions d'éclairement minimal la vision de loin soit privilégiée.

Compte tenu de la variabilité de la détermination statistique des diamètres d'ouverture pupillaire et de la valeur relative, également variable, des diamètres pupillaires de référence (2, 2,8 et 3,5 mm), les fourchettes pratiques sont, en réalité, légèrement plus larges et telles que :

$$0,8 \text{ mm} \leqq d_1 \leqq 1,6 \text{ mm}$$

$$1,4 \text{ mm} \leqq d_2 \leqq 2,8 \text{ mm}$$

$$2,4 \text{ mm} \leqq d_3 \leqq 3,6 \text{ mm}$$

Avantageusement, il s'avère que l'on obtient de bons résultats lorsqu'en condition d'éclairement maximum ou minimum, la surface consacrée à la vision de loin atteint au moins 60 % de la surface totale éclairée pour au moins un diamètre d'ouverture pupillaire. De manière similaire, en condition d'éclairement intermédiaire, la surface consacrée à la vision de près doit atteindre au moins 60 % de la surface totale éclairée. De telles conditions, un peu plus restrictives, assurent des conditions parfaitement reproductibles de vision privilégiée, de près ou de loin, dans toutes les conditions d'éclairement.

Il doit, également, être noté que, pour permettre une vision bifocale utile pratiquement, la surface consacrée à la vision de près doit, avantageusement, toujours représenter au moins 25 % de la surface totale éclairée en condition d'éclairement maximum et minimum. Corrélativement, la surface consacrée à la vision de loin doit représenter au moins 25 % de la surface totale éclairée en condition d'éclairement intermédiaire.

Il est bien évident que, parmi les fourchettes de dimensions de zones données précédemment, différentes combinaisons de surface de zones sont possibles sans sortir du cadre de l'invention, dès l'instant où les rapports précédents, entre la surface totale éclairée et la surface consacrée respectivement à la vision de près et de loin, sont respectées. A titre purement indicatif, on obtient de bons résultats avec un implant intra-oculaire selon l'invention comportant une première zone **13** d'un diamètre **d** de 1,1 mm, associée à une seconde zone **14** d'un diamètre extérieur de 2,2 mm, associée à une troisième zone **15** d'un diamètre extérieur de 3,2 mm.

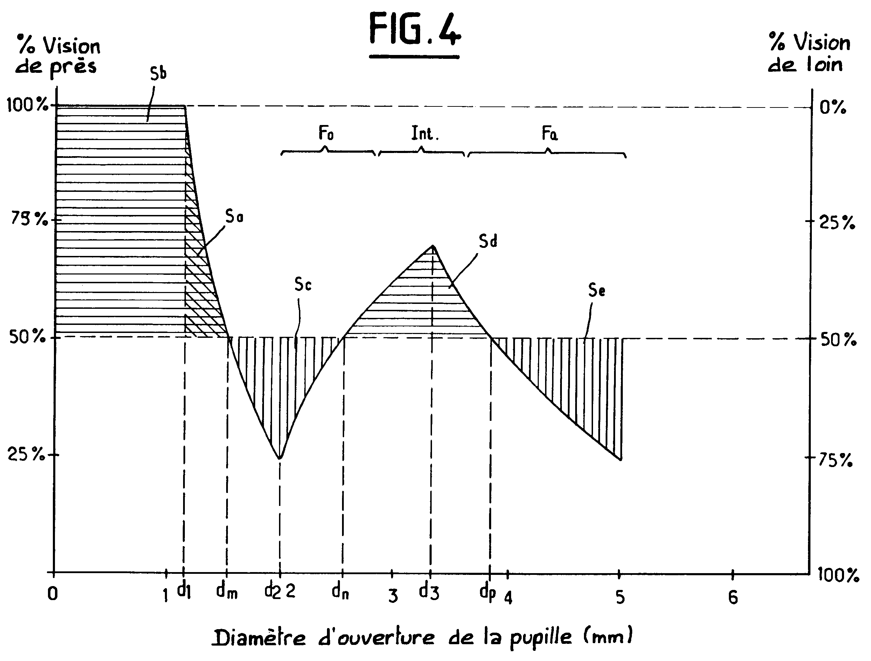

On a représenté, à la **fig. 4**, un graphe montrant les particularités de transmission lumineuse caractéristiques des implants conformes à l'invention. Le graphe de la **fig. 4** est spécifique d'un implant possédant une répartition de ses zones de vision de près et de loin données, à savoir une zone centrale **13** de vision de près d'un diamètre $d_1$ voisin de 1,1 mm, une seconde zone **14** de vision de loin d'un diamètre extérieur $d_2$ voisin de 1,95 mm, une troisième zone annulaire **15** de vision de près d'un diamètre $d_3$ extérieur voisin de 3,4 mm.

Sur le graphe de la **fig. 4**, on a représenté en abscisse le diamètre moyen d'ouverture d'une pupille (en millimètres) et on a représenté, sur les axes d'ordonnée le pourcentage de vision de près et le pourcentage de vision de loin correspondant au pourcentage de la surface des diverses zones de l'implant éclairées à travers la pupille et consacrées, respectivement, à la vision de près et de loin.

On voit que, dans les conditions d'éclairement maximum correspondant à une pupille rétractée d'un diamètre voisin de 2 mm, les implants selon l'invention permettent, dès que la surface totale éclairée de l'implant dépasse le diamètre $d_1$, une vision bifocale de près et de loin représentée par la surface **Sa**, la surface **Sb** ne présentant pas d'intérêt pratique et ne correspondant qu'à une situation théorique. Lorsque l'intensité lumineuse diminue, la pupille se dilate et l'implant va permettre progressivement, dans la zone correspondant à la surface **Sa**, de priviligier de manière décroissante la vision de près jusqu'à la valeur de référence $d_m$ associée à une vision équivalente de près et de loin (50-50).

Dans les conditions d'éclairement maximales, référencées **Fo** sur la **fig. 4**, correspondant à des données pratiques de forte luminosité pour lesquelles le diamètre d'ouverture de la pupille est compris dans une plage variant de 2 mm à un peu moins de 3 mm, l'implant étudié présente une zone de vision caractéristique représentée par la surface **Sc** privilégiant la vision de loin, puisque, dans tous les cas, la surface des zones réservées à la vision de loin est supérieure à 50 % de la surface totale éclairée.

La vision de loin est privilégiée au maximum lorsque la pupille possède une ouverture proche du diamètre $d_2$ correspondant à une répartition de l'intensité lumineuse à 75 % pour la vision de loin et à 25 % pour la vision de prés. Pour les autres conditions de forte luminosité, associées à un diamètre d'ouverture des pupilles au-delà de $d_2$, la vision de loin sera encore privilégiée jusqu'à une autre valeur spécifique $d_n$ au-delà de laquelle la vision de prés est alors privilégiée, puisque plus de 50 % de la surface totale éclairée lui est consacré.

Cette particularité est représentée, sur le graphe de la **fig. 4**, par la surface **Sd**. Cette dernière chevauche les conditions de luminosité dites maximales et intermédiaires, ces dernières, référencées **Int** sur la **fig.**

**4** étant généralement associées à un diamètre d'ouverture pupillaire compris entre 2,8 et 3,5 mm. Dans ces conditions d'éclairement intermédiaire, réservées normalement à l'opération de lecture, l'observateur aura, dans tous les cas, la possibilité de voir de près et de loin, mais l'implant selon l'invention privilégiera la vision de près dont les conditions de vision de près sont maximales pour une ouverture pupillaire correspondant à **d₃** et proche de 65 % (vision de près).

Il doit également être noté que, dans les conditions de faible éclairement **Fo** ou d'éclairement minimal associées à un diamètre pupillaire compris entre 3,5 et 5 mm, les implants selon l'invention permettent, progressivement, lorsque la luminosité décroit, de passer d'une situation dans laquelle la vision de près est privilégiée à une situation dans la zone **Se** au-delà du diamètre **dₚ** où la vision de loin est progressivement privilégiée.

Il est bien évident que le graphe, représenté à la **fig. 4**, est spécifique des caractéristiques dimensionnelles d'un implant et que lorsque ces dernières varient, les valeurs caractéristiques des zones **Sa**, **Sc** et **Sd** évoluent corrélativement. Le principe de base, consistant à privilégier, en condition d'éclairement maximum et minimum, la vision de loin et, en condition d'éclairement intermédiaire, la vision de près est inchangé, le graphe étant simplement décalé ou présentant des pics caractéristiques différents.

La réalisation d'un implant conforme à l'invention, présentant les trois zones de vision alternée, telles que décrites précédemment dans les rapports de surface également mentionnés précédemment, permet à un observateur utilisant cet implant de s'adapter en toute circonstance aux variations d'intensité lumineuse et de conserver une vision de près et de loin privilégiant les activités essentielles de l'activité humaine. L'implant selon l'invention permet, également, d'assurer une plus grande sécurité de mouvement ou de conduite des personnes en permettant, même en cas de très fort éblouissement, à l'observateur une vision de loin privilégiée, tout en privilégiant, également, en cas de faible luminosité, la vision de loin. Un tel implant s'accommode donc de conditions particulièrement extrêmes d'éclairement.

Il est également possible d'utiliser le principe de disposition et de rapport de surface des zones de vision de près et de loin sur tout dispositif optique adapté à la correction de vision et, en particulier, par exemple, sur des lentilles ou verres de contact.

De la même façon, l'implant selon l'invention peut comporter plus de deux focales et posséder un pouvoir de correction plus ou moins élevé.

L'invention n'est pas limitée aux exemples décrits et représentés, car diverses modifications peuvent y être apportées sans sortir de son cadre.

**Revendications**

1.  Implant intra-oculaire bifocal rétractif comportant un bloc optique (**11**) transparent consistant en une zone centrale (**13**) de diamètre $d_1$ et des zones annulaires/concentriques (**14,15,16**) de diamètres respectifs $d_2$, $d_3$ et $d_4$, de correction de vision, chaque zone étant, à partir de la zone centrale (**13**) réservée à la vision de près, alternativement, une zone de vision de loin (**14**) et de près (**15**),
    caractérisée en ce que les surfaces respectives (**s₁, s₂**) de chacune des zones (**13, 14**) sont inégales en ce qu'il ne comprend que trois zones annulaires concentriques (**14,15,16**) en ce que les diamètres $d_1$, $d_2$ et $d_3$ verifient les relations suivantes :

    $d_1$ est compris entre 0,8 et 1,6 mm
    $d_2$ est compris entre 1,4 et 2,8 mm et
    $d_3$ est compris entre 2,4 et 3,6 mm, et et en ce que; dans des conditions d'éclairement

    maximum (Fo) ou minimum (Fa), la surface consacrée á la vision de loin atteint au moins 60% de la surface totale éclairée.

2.  Implant selon la revendication 1, caractérisée en ce qu'elle comporte quatre zones (**13**, **14**, **15**, **16**) de correction de vision alternées et en ce que les surfaces respectives des zones (**s₁**, **s₂**, **s₃**, **s₄**) sont choisies de telle manière que, dans des conditions d'éclairement intermédiaire (**Int**), la surface consacrée à la vision de prés est supérieure à la moitié de la surface totale éclairée.

3.  Implant selon la revendication 2, caractérisée en ce que, en condition d'éclairement intermédiaire (**Int**), la surface consacrée à la vision de près atteint au moins 60 % de la surface totale éclairée.

4.  Implant selon l'une des revendications précédentes, caractérisée en ce que, en condition d'éclairement maximum (**Fo**) ou minimum (**Fa**), la surface consacrée à la vision de prés représente au moins 25 % de la surface toale éclairée.

5.  Implant selon l'une des revendications précédentes, caractérisée en ce que, en condition d'éclairement intermédiaire (**Int**), la surface consacrée à la vision de loin représente au moins 25 % de la surface totale éclairée.

6.  Implant selon l'une des revendications précédentes, caractérisée en ce que :

    -   la zone centrale (**13**) de vision de près a un diamètre (**d₁**) voisin de 1,1 mm,
    -   la seconde zone adjacente (**14**) de vision de

- loin a un diamètre (**d₂**) voisin de 2,2 mm,
- la troisième zone adjacente (**15**) de vision de près a un diamètre (**d₃**) voisin de 3,2 mm.

## Claims

1. Retractive, bifocal intraocular implant comprising a transparent optical unit (11) which consists of a central zone (13) for correcting vision having a diameter $d_1$ and concentric annular zones (14, 15, 16) for correcting vision having respective diameters $d_2$, $d_3$ and $d_4$, each zone being, from the central zone (13) reserved to near vision, alternately a zone of distance vision (14) and of near vision (15), **characterised in that** the respective surfaces ($s_1$, $s_2$) of each of the zones (13,14) are unequal, in that it only comprises three annular concentric zones (14,15,16), in that the diameters $d_1$, $d_2$ and $d_3$ confirm the following relationships:

   $d_1$ is between 0.8 and 1.6 mm;
   $d_2$ is between 1.4 and 2.8 mm and
   $d_3$ is between 2.4 and 3.6 mm

   and in that, in conditions of maximum (Fo) or minimum (Fa) illumination, the surface dedicated to distance vision attains at least 60% of the total illuminated surface.

2. Implant according to claim 1, **characterised in that** it comprises four alternate zones (13, 14, 15, 16) for correcting vision and in that the respective surfaces of the zones ($s_1$, $s_2$, $s_3$, $s_4$) are selected in such a way that, in conditions of intermediate illumination (Int), the surface dedicated to near vision is greater than half the total illuminated surface.

3. Implant according to claim 2, **characterised in that,** in a condition of intermediate illumination (Int), the surface dedicated to near vision attains at least 60% of the total illuminated surface.

4. Implant according to one of the preceding claims, **characterised in that,** in a condition of maximum (Fo) or minimum (Fa) illumination, the surface dedicated to near vision represents at least 25% of the total illuminated surface.

5. Implant according to one of the preceding claims, **characterised in that,** in a condition of intermediate illumination (Int), the surface dedicated to distance vision represents at least 25% of the total illuminated surface.

6. Implant according to one of the preceding claims, **characterised in that:**

- the central zone (13) of near vision has a diameter ($d_1$) close to 1.1 mm,
- the second adjacent zone (14) of distance vision has a diameter ($d_2$) close to 2.2 mm,
- the third adjacent zone (15) of near vision has a diameter ($d_3$) close to 3.2 mm.

## Patentansprüche

1. Intraokulares, bifokales, retraktives Implantat, enthaltend einen transparenten, optischen Block (11), der aus einem Mittelbereich (13) mit einem Durchmesser $d_1$ und aus ringförmigen, konzentrischen Bereichen (14,15,16) mit jeweiligen Durchmessern $d_2$, $d_3$ und $d_4$ besteht, zur Sehkorrektur, wobei jeder Bereich ausgehend von dem der Nahsicht vorbehaltenen Mittelbereich (13) abwechselnd ein Bereich zur Weitsicht (14) und ein Bereich zur Nahsicht (15) ist, dadurch gekennzeichnet, daß die jeweiligen Flächen ($s_1$, $s_2$) eines jeden Bereichs (13, 14) ungleich sind, daß es nur drei ringförmige, konzentrische Bereiche (14, 15, 16) enthält, daß die Durchmesser $d_1$, $d_2$ und $d_3$ folgende Bedingungen erfüllen:

   $d_1$ liegt zwischen 0,8 und 1,6 mm,
   $d_2$ liegt zwischen 1,4 und 2,8 mm, und
   $d_3$ liegt zwischen 2,4 und 3,6 mm,

   und daß die der Weitsicht vorbehaltene Fläche bei maximalen (Fo) oder minimalen (Fa) Lichtverhältnissen mindestens 60 % der insgesamt beleuchteten Fläche erreicht.

2. Implantat nach Anspruch 1, dadurch gekennzeichnet, daß es vier abwechselnde Bereiche (13, 14, 15, 16) zur Sehkorrektur enthält, und daß die jeweiligen Flächen ($s_1$, $s_2$, $s_3$, $s_4$) der Bereiche so bemessen sind, daß bei mittleren Lichtverhältnissen (Int) die der Nahsicht vorbehaltene Fläche größer ist als die Hälfte der insgesamt beleuchteten Fläche.

3. Implantat nach Anspruch 2, dadurch gekennzeichnet, daß bei mittleren Lichtverhältnissen (Int) die der Nahsicht vorbehaltene Fläche mindestens 60 % der insgesamt beleuchteten Fläche erreicht.

4. Implantat nach einem der vorhergehenden Ansprüche, dadurch gekennzeichnet, daß bei maximalen (Fo) oder bei minimalen (Fa) Lichtverhältnissen die der Nahsicht vorbehaltene Fläche mindestens 25 % der insgesamt beleuchteten Fläche darstellt.

5. Implantat nach einem der vorhergehenden Ansprüche, dadurch gekennzeichnet, daß bei mittleren Lichtverhältnissen (Int) die der Weitsicht vorbehaltene Fläche mindestens 25 % der insgesamt beleuchteten Fläche darstellt.

6. Implantat nach einem der vorhergehenden Ansprüche, dadurch gekennzeichnet, dass

- der Mittelbereich (13) zur Nahsicht einen Durchmesser ($d_1$) von etwa 1,1 mm aufweist,
- der daran anschließende zweite Bereich (14) zur Weitsicht einen Durchmesser ($d_2$) von etwa 2,2 mm aufweist,
- der daran anschließende dritte Bereich (15) zur Nahsicht einen Durchmesser ($d_3$) von etwa 3,2 mm aufweist.

**FIG.1**

**FIG.2**

**FIG.3**

EP 0 488 835 B1

# FIG.4

% Vision de près

% Vision de loin

100% — 0%

Sb

Fo    Int.    Fa

75% — 25%

Sa

Sd

Sc

Se

50% — 50%

25% — 75%

100%

0    1  d1  dm  d2 2  dn   3  d3  dp 4    5    6

Diamètre d'ouverture de la pupille (mm)

EP 0 488 835 B1